# EUROPEAN PATENT APPLICATION

(11) **EP 4 779 037 A1**
(43) Date of publication of application: **22.07.2026**
(21) Application number: 25177036.8
(22) Date of filing: 16.05.2025
(51) Int. Cl.: C13K 1/02

(54) **CELLULOSE PRETREATMENT AGENT AND APPLICATIONS THEREOF IN ENZYMATIC HYDROLYSIS OF CELLULOSE-CONTAINING BIOMASS**

(30) Priority: 20.01.2025 CN 202510085864; 18.04.2025 US 202519182658
(71) Applicant: Hong Kong Applied Science and Technology Research Institute Company Limited, Hong Kong (HK)
(72) Inventor: CHAU, Wing Yu, Shatin, New Territories, Hong Kong (CN); CHAN, Wing Lam, Shatin, New Territories, Hong Kong (CN); YIP, Hang Kuen, Shatin, New Territories, Hong Kong (CN); MIAO, Yanan, Shatin, New Territories, Hong Kong (CN); SOECIPTO, Aristyo, Shatin, New Territories, Hong Kong (CN); KO, Pui Kei, Shatin, New Territories, Hong Kong (CN); FU, Qijia, Shatin, New Territories, Hong Kong (CN)
(74) Representative: ABG Intellectual Property Law, S.L.

(57) **Abstract**

The present disclosure provides a cellulose pretreatment agent, a method for pretreating cellulose with the cellulose pretreatment agent, and a method for producing a reducing sugar. The cellulose pretreatment agent is an organic acid comprising at least one sulfonic acid group, a carbon-based material derived from the organic acid comprising at least one sulfonic acid group, or a g-C₃N₄/FeOCl Fenton-like composite. The pretreatment of the present disclosure can be performed under a mild condition and the cellulose-containing biomass pretreated with the cellulose pretreatment agent of the present disclosure can be enzymatically hydrolyzed under a mild condition, with less hazardous chemicals used.

## Description

### CROSS-REFERENCE TO RELATED APPLICATION

This application claims the priority benefit of China application serial no. 2025100858646, filed on January 20, 2025. The entirety of the above-mentioned patent application is hereby incorporated by reference herein and made a part of this specification.

### TECHNICAL FIELD

The present disclosure relates to the technical field of catalysis/material science. More particularly, it relates to a cellulose pretreatment agent and applications thereof in enzymatic hydrolysis of cellulose-containing biomass.

### BACKGROUND

In recent years, the concept of "carbon neutrality" has gained significant emphasis as a broader commitment to decarbonization and climate action within the chemical and biochemical processing sectors. Biomass conversion has emerged as a vital method for various applications, including energy and chemical production. The feedstock for biomass conversion, which is normally waste material containing cellulose, undergoes initial conversion through pretreatment and enzymatic hydrolysis into intermediates such as reducing sugars and amorphous bio-mixtures, which is subsequently followed by additional chemical or biological processing to yield the final potential products.

The conversion of cellulose-containing biomass involves a pre-treatment step that is often the most difficult to navigate. Generally, biomass is made up of 40-50% cellulose, 25-30% hemicellulose, and 15-20% lignin, with these proportions varying by source. The presence of hemicellulose and lignin creates barriers that limit cellulose accessibility during enzymatic hydrolysis, while the crystalline structure of cellulose further complicates the process. The goal of pretreatment is to dismantle these barriers by breaking down the hydrogen bonding network and van der Waals forces associated with lignin and hemicellulose. After successful pretreatment, enzymatic hydrolysis can be performed under mild conditions, which significantly improves the conversion yield to intermediates.

The traditional textile recycling process faces several challenges beyond the initial pretreatment stage. Issues related to sorting, decolorization, and the quality of intermediate products hinder the widespread adoption of textile recycling. Most textiles today are made from blended fabrics, necessitating the sorting of crushed materials into different recycling streams. This separation process is complex, resulting in inefficiencies in subsequent recycling stages. Additionally, after the enzymatic hydrolysis, residual textile dyes persist, making decolorization through active carbon columns a critical step. Ultimately, the fibers produced from these recycling efforts are predominantly short, limiting their application to low-value products.

Thus, there is a need in the art for a method for pretreating cellulose-containing biomass, so that the resulting cellulose-containing biomass can be enzymatically hydrolyzed under a mild reaction condition, with less hazardous chemicals used.

### SUMMARY

The present disclosure seeks to provide a method for the conversion of cellulose-containing biomass under a mild and environmentally-friendly reaction condition. The inventors have developed a cellulose pretreatment agent. The cellulose-containing biomass pretreated with the cellulose pretreatment agent can be enzymatically hydrolyzed under a relatively mild reaction condition, with less hazardous chemicals used, thereby achieving the present invention.

In a first aspect of the present disclosure, provided is a cellulose pretreatment agent, wherein the cellulose pretreatment agent is an organic acid comprising at least one sulfonic acid group or a carbon-based material derived from the organic acid comprising at least one sulfonic acid group.

In a second aspect of the present disclosure, provided is a method for pretreating cellulose, comprising a step of (1) pretreating cellulose-containing biomass with a cellulose pretreatment agent, wherein the cellulose pretreatment agent is an organic acid comprising at least one sulfonic acid group, a carbon-based material derived from the organic acid comprising at least one sulfonic acid group, or a g-C₃N₄/FeOCl Fenton composite.

In a third aspect of the present disclosure, provided is a method for producing a reducing sugar, comprising steps of:
(1) pretreating cellulose-containing biomass with a cellulose pretreatment agent, wherein the cellulose pretreatment agent is an organic acid comprising at least one sulfonic acid group, a carbon-based material derived from the organic acid comprising at least one sulfonic acid group, or a g-C₃N₄/FeOCl Fenton-like composite;
(2) separating the pretreated cellulose-containing biomass from the cellulose pretreatment agent; and
(3) subjecting the separated and pretreated cellulose-containing biomass to enzymatic hydrolysis.

In a fourth aspect of the present disclosure, provided is use of the cellulose pretreatment agent according to the first aspect or a g-C₃N₄/FeOCl Fenton composite for pretreating cellulose-containing biomass.

The cellulose-containing biomass, such as fabrics, e.g. cotton fabrics or polyester/cotton blended fabrics, pretreated with the cellulose pretreatment agent according to the present disclosure, can be enzymatically hydrolyzed under a relatively mild reaction condition, with less hazardous chemicals used. Said relatively mild reaction condition comprises, e.g. a relatively low temperature such as from 40°C to 60°C for enzymatic hydrolysis; no need to use any acid, base, inducer or ionic liquid; and/or use of a more neutral reaction system. The cellulose-containing biomass pretreated with the cellulose pretreatment agent according to the present disclosure can be enzymatically hydrolyzed more efficiently, manifesting as either an increased yield of the reducing sugar obtained from the fabrics by using the same amount of enzymes or a similar yield of the reducing sugar by using a less amount of enzymes. The cellulose-containing biomass pretreated with the cellulose pretreatment agent according to the present disclosure, when being subjected to enzymatic hydrolysis, may produce the reducing sugar at a yield of higher than 80%.

### BRIEF DESCRIPTION OF THE DRAWINGS

The accompanying drawings are included to provide a further understanding of the disclosure and are incorporated in and constitute a part of this specification. The drawings described herein are for illustration purposes only and are not intended to limit the scope of the disclosure in any way.
FIG. 1 illustrates the infrared spectra of tannic acid, sulfomethyl tannic acid, carbon quantum dots and carbon black of the present disclosure.
FIG. 2 illustrates the transmission electron microscope (TEM) images of the carbon quantum dots prepared from citric acid and sulfomethyl tannic acid of the present disclosure.
FIG. 3 illustrates the UV-Vis absorption spectra and photoluminescence fluorescence spectra of the carbon quantum dots prepared from citric acid and sulfomethyl tannic acid of the present disclosure.
FIG. 4 illustrates the photographs of the carbon quantum dots prepared from citric acid and sulfomethyl tannic acid of the present disclosure under daylight (left panel) and under 365 nm excitation (right panel).
FIG. 5 illustrates the photographs of the carbon quantum dots prepared from citric acid and metanilic acid of the present disclosure under daylight (left panel) and under 365 nm excitation (right panel).
FIG. 6 illustrates the photographs of the pretreated mixture containing blue fabrics before (left panel) and after (right panel) pretreatment.
FIG. 7 illustrates the photographs of the fabrics washed with deionized water and oven-dried at 70°C.
FIG. 8 illustrates the schematic diagram of the cellulose pretreatment agent of the present disclosure.

### DESCRIPTION OF THE EMBODIMENTS

The following detailed description is merely exemplary in nature and is in no way intended to limit the present disclosure or its application or uses.

Notwithstanding that the numerical ranges and parameters setting forth the broad scope of the present disclosure are approximations, the numerical values set forth in the specific examples are reported as precisely as possible. Any numerical value *per se,* however, inherently contains certain errors, e.g. within ±5%, necessarily resulted from the standard variation found in their respective testing measurements.

Also, it should be understood that any numerical range recited herein is intended to include end values, and all sub-ranges and values subsumed therein. For example, a numerical range of " 1 to 10" is intended to include the minimum value of 1 and the maximum value of 10, and all sub-ranges subsumed therein, that is, the minimum value may be equal to or greater than 1, and the maximum value may be equal to or less than 10.

The terminology used herein is for the purpose of describing particular exemplified embodiments only and is not intended to be limiting.

As used herein, the articles "a", "an", and "the", and the expressions "at least one" and "one or more" are used interchangeably to indicate that at least one of the specified elements, materials, ingredients or method steps is present, unless the context clearly indicates otherwise.

As used herein, the terms "about", "substantially" and "essentially" are used herein with respect to measurable values and ranges due to expected variations known to those skilled in the art (e.g., limitations and variability in measurements). The term "about" also indicates that the stated numerical value allows some slight imprecision (with some approach to exactness in the value; approximately or reasonably close to the value; nearly). If the imprecision provided by the term "about" is not otherwise understood in the art with this ordinary meaning, then the term "about" as used herein indicates at least variations that may arise from ordinary methods of measuring and using such parameters. In addition, disclosure of ranges includes disclosure of all values and further divided ranges within the entire range.

As used herein, the terms "including", "containing" and the like terms, together with their grammatical variations, are synonymous with the term "comprising" and its grammatical variations. The term "comprising" and its grammatical variations are open-ended and shall be understood in the context of the present disclosure to include not only the specified elements, materials, ingredients or method steps, but also additional unspecified elements, materials, ingredients or method steps, provided that the additional unspecified elements, materials, ingredients or method steps do not have contradictory or contrary meanings to the specified elements, materials, ingredients or method steps.

When specific aspects of the present disclosure are described in detail, it will be appreciated by those skilled in the art that various modifications and alternatives to those details could be developed in light of the overall teachings of the present disclosure. Accordingly, the particular arrangements disclosed are meant to be illustrative only, but not limiting as to the scope of the disclosure, and the scope of the disclosure shall include the full scope of the claims appended and any and all equivalents thereof.

As mentioned above, the present disclosure aims to provide a method for the conversion of cellulose-containing biomass under a mild and environmentally-friendly reaction condition.

In a first aspect of the present disclosure, provided is a cellulose pretreatment agent, wherein the cellulose pretreatment agent is an organic acid comprising at least one sulfonic acid group or a carbon-based material derived from the organic acid comprising at least one sulfonic acid group.

In the context of the present disclosure, the term "pretreatment", "pretreating" or any other grammatical variation refers to the thermal and/or chemical treatment of cellulose-containing biomass materials prior to enzymatic hydrolysis by cellulase.

In the context of the present disclosure, the term "organic acid comprising at least one sulfonic acid group" may refer to an organic acid which itself contains a sulfonic acid group, or an organic acid which itself contains no sulfonic acid group, but is modified to contain at least one sulfonic acid group. In an organic acid modified with at least one sulfonic acid group, the sulfonic acid group is incorporated via one or more, preferably more, hydroxyl groups or other reactive groups on the organic acid that can be substituted with the sulfonic acid group. It should be understood that the sulfonic acid group may completely or partially replace the hydroxyl groups or the reactive groups on the organic acid, which is not particularly defined in the present disclosure, as long as the resultant product is able to effectively pretreat the cellulose-containing biomass.

In a particular embodiment, the organic acid comprising at least one sulfonic acid group may be, but is not limited to, sulfonic tannic acid and/or metanilic acid.

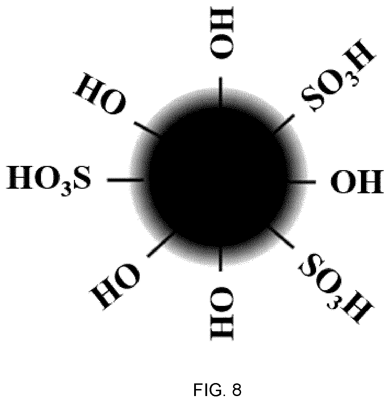

In the context of the present disclosure, the term "carbon-based material" may refer to a support that is formed mainly from carbon material and can be loaded with active ingredients including substituents, catalysts, and the like. The inventors have found that the carbon-based material prepared from the organic acid comprising at least one sulfonic acid group of the present disclosure retains the sulfonic acid group thereon, forming a structure in which the carbon-based material constitutes the center particle, and the sulfonic acid group and the hydroxyl group or other reactive group, if present, are located on the surface of the center particle, as shown in FIG. 8.

Due to the presence of the sulfonic acid group and the hydroxyl or reactive group (in some cases) on the cellulose pretreatment agent of the present disclosure, the cellulose pretreatment agent of the present disclosure can undergo sufficient chemical reaction with the cellulose-containing biomass under a mild condition, which allows to loosen the cellulose structure and reduce crystallinity degree and the polymerization degree of the cellulose, thereby promoting subsequent enzymatic hydrolysis by cellulase to be carried out smoothly under a relatively mild condition.

In another particular embodiment, the carbon-based material derived from the organic acid comprising at least one sulfonic acid group may be, but is not limited to, carbon black or carbon quantum dots. For example, the carbon-based material may be carbon black or carbon quantum dots prepared by reacting an organic acid comprising at least one sulfonic acid group, such as sulfonic tannic acid and/or metanilic acid, with citric acid.

In the context of the present disclosure, the term "carbon quantum dots (CQDs)", also known as carbon dots or carbon nanodots, is a quantum structure consisting of ultrafine, dispersed, quasi-spherical and nanoscale carbon nanoparticles with a particle size of less than 10 nm. In the present disclosure, the carbon quantum dots may have a particle size of less than 10 nm, for example having a particle size in the range of 2 nm - 5 nm.

The cellulose pretreatment agent of the present disclosure is soluble in water when in the form of an organic acid comprising at least one sulfonic acid group and insoluble in water when in the form of a carbon-based material. Thus, the cellulose pretreatment agent of the present disclosure can be selected according to the requirements of subsequent applications, e.g. whether it is desired to recover the cellulose pretreatment agent.

In another particular embodiment, the cellulose pretreatment agent may be a sulfonic tannic acid with a sulfonic acid group content of 1 mmol/g - 4 mmol/g. For example, the sulfonic acid group content in the sulfonic tannic acid may be 1 mmol/g, 1.25 mmol/g, 1.5 mmol/g, 1.75 mmol/g, 2 mmol/g, 2.25 mmol/g, 2.5 mmol/g, 2.75 mmol/g, 3 mmol/g, 3.25 mmol/g, 3.5 mmol/g, 3.75 mmol/g or 4 mmol/g or in any range composed of the above numerical values. In a preferred embodiment, the cellulose pretreatment agent may be a sulfonic tannic acid with a sulfonic acid group content of 1.5 mmol/g - 2 mmol/g.

In another particular embodiment, the sulfonic tannic acid may be, but is not limited to a sulfomethyl tannic acid.

In a second aspect of the present disclosure, provided is a method for pretreating cellulose, comprising a step of (1) pretreating cellulose-containing biomass with a cellulose pretreatment agent, wherein the cellulose pretreatment agent is an organic acid comprising at least one sulfonic acid group, a carbon-based material derived from the organic acid comprising at least one sulfonic acid group, or a g-C₃N₄/FeOCl Fenton-like composite.

In a particular embodiment, the organic acid comprising at least one sulfonic acid group may be, but is not limited to, a sulfonic tannic acid and/or a metanilic acid.

In a particular embodiment, the carbon-based material derived from the organic acid comprising at least one sulfonic acid group may be, but is not limited to, carbon black or carbon quantum dots. For example, the carbon-based material may be carbon black or carbon quantum dots prepared by reacting an organic acid comprising at least one sulfonic acid group, such as sulfonic tannic acid and/or metanilic acid, with citric acid.

In another particular embodiment, the cellulose pretreatment agent may be a sulfonic tannic acid with a sulfonic acid group content of 1 mmol/g - 4 mmol/g. For example, the sulfonic acid group content in the sulfonic tannic acid may be 1 mmol/g, 1.25 mmol/g, 1.5 mmol/g, 1.75 mmol/g, 2 mmol/g, 2.25 mmol/g, 2.5 mmol/g, 2.75 mmol/g, 3 mmol/g, 3.25 mmol/g, 3.5 mmol/g, 3.75 mmol/g or 4 mmol/g or in any range composed of the above numerical values.

In a preferred embodiment, the cellulose pretreatment agent may be a sulfonic tannic acid with a sulfonic acid group content of 1.5 mmol/g - 2 mmol/g.

In another particular embodiment, the sulfonic tannic acid may be, but is not limited to a sulfomethyl tannic acid.

It should be understood that the above description of the cellulose pretreatment agent according to the first aspect of the present disclosure is also applicable to the cellulose pretreatment agent used in the method for pretreating cellulose according to the second aspect of the present disclosure. Thus, such content will not be repeated herein for the sake of brevity.

In another particular embodiment, the cellulose pretreatment agent is a g-C₃N₄/FeOCl Fenton-like composite. The g-C₃N₄/FeOCl Fenton composite may be used to pretreat colored cellulose-containing biomass, so as to efficiently de-color the cellulose-containing biomass.

In the context of the present disclosure, the term "Fenton-like composite" refers to materials containing multivalent metals such as Fe, Cu, Co, Mn, Ce, Ag, Cr, Ru, W, Mo, V, Ti, etc., which can activate peroxide such as H₂O₂, potassium peroxymonosulfate, etc., and generate reactive oxygen species (ROS) through a low valence of a metal (Mⁿ⁺) with reducing property and a high valence of the metal (M^{(n+z)+}) with oxidizing property. Taking metal Fe as an example, the low valence is Fe²⁺ and the high valence is Fe³⁺, so that n is 2 and z is 1.

As used herein, the term "cellulose-containing biomass" refers to biomass prepared from plant material such as wood, straw, corn stalks and hemp plants.

In a particular embodiment, the Cellulose-containing biomass is fabrics, e.g. cotton fabrics, polyester/cotton blended fabrics and the like.

In a particular embodiment, step (1) comprises heating a mixture of the pretreatment agent and the cellulose-containing biomass to a temperature, e.g., from 40°C to 60°C, for a period of time. Preferably, the heating is performed with shaking or stirring, to promote well mixing of the cellulose pretreatment agent with the biomass and temperature uniformity throughout the reaction system.

The cellulose pretreatment in the prior art is typically performed at a relatively high temperature, e.g., from 90°C to 120°C. In contrast, the cellulose pretreatment according to the present disclosure can be performed at a relatively mild temperature.

In a preferred embodiment, step (1) comprises heating the mixture of the cellulose pretreatment agent and the cellulose-containing biomass to a temperature from 45°C to 55°C.

In a more preferred embodiment, step (1) comprises heating the mixture of the pretreatment agent and the cellulose-containing biomass to a temperature of 50°C.

In another particular embodiment, the mixture of the pretreatment agent and the cellulose-containing biomass may be maintained at the above-mentioned temperature for 12 h - 48 h, e.g., 24 h.

Furthermore, an acidic solution, an alkaline solution, or an ionic liquid is usually used as a solvent in the method of pretreating cellulose in the prior art. However, in the cellulose pretreatment according to the present disclosure, water can be used as a solvent with no need to use any acidic/alkaline solution or ionic liquid. As a result, the cost issues associated with acid and base recovery and waste disposal become moot, and there is no need to use any corrosion-resistant container.

Thus, in a particular embodiment, step (1) comprises adding the cellulose pretreatment agent and the cellulose-containing biomass into water to form a mixture of the cellulose pretreatment agent and the cellulose-containing biomass, and then heating the mixture.

In another particular embodiment, in step (1), the cellulose pretreatment agent is at a concentration of 0.5 g/L to 20 g/L. For example, the cellulose pretreatment agent may be at a concentration of 0.5 g/L, 1 g/L, 1.5 g/L, 2 g/L, 2.5 g/L, 3 g/L, 3.5 g/L, 4 g/L, 4.5 g/L, 5 g/L, 5.5 g/L, 6 g/L, 6.5 g/L, 7 g/L, 7.5 g/L, 8 g/L, 8.5 g/L, 9 g/L, 9.5 g/L, 10 g/L, 10.5 g/L, 11 g/L, 11.5 g/L, 12 g/L, 12.5 g/L, 13 g/L, 13.5 g/L, 14 g/L, 14.5 g/L, 15 g/L, 15.5 g/L, 16 g/L, 16.5 g/L, 17 g/L, 17.5 g/L, 18 g/L, 18.5 g/L, 19 g/L, 19.5 g/L or 20 g/L, or in any range composed of the above numerical values.

In a particular embodiment, in step (1), the g-C₃N₄/FeOCl Fenton-like composite is at a concentration of 1 g/L to 3 g/L. For example, the g-C₃N₄/FeOCl Fenton-like composite may be at a concentration of 1 g/L, 1.5 g/L, 2 g/L, 2.5 g/L or 3 g/L, or in any range composed of the above numerical values.

It should be understood that the cellulose-containing biomass may be milled into fine powder before it is pretreated with the cellulose pretreatment agent, to increase the contact area between the cellulose pretreatment agent and the cellulose-containing biomass, and to promote the pretreatment effect of the cellulose pretreatment agent on the cellulose-containing biomass. However, from the perspective of subsequent separation of the cellulose pretreatment agent from the cellulose-containing biomass, it is preferred not to mill the cellulose-containing biomass into fine powder before the pretreatment.

In a third aspect of the present disclosure, provided is a method for producing a reducing sugar, comprising steps of:
(1) pretreating cellulose-containing biomass with a cellulose pretreatment agent, wherein the cellulose pretreatment agent is an organic acid comprising at least one sulfonic acid group, a carbon-based material derived from the organic acid comprising at least one sulfonic acid group, or a g-C₃N₄/FeOCl Fenton-like composite;
(2) separating the pretreated cellulose-containing biomass from the cellulose pretreatment agent; and
(3) subjecting the separated and pretreated cellulose-containing biomass to enzymatic hydrolysis.

In a particular embodiment, the organic acid comprising at least one sulfonic acid group may be, but is not limited to a sulfonic tannic acid and/or a metanilic acid.

In a particular embodiment, the carbon-based material derived from the organic acid comprising at least one sulfonic acid group may be, but is not limited to, carbon black or carbon quantum dots. For example, the carbon-based material may be carbon black or carbon quantum dots prepared by reacting an organic acid comprising at least one sulfonic acid group, such as a sulfonic tannic acid and/or a metanilic acid, with a citric acid.

In another particular embodiment, the cellulose pretreatment agent may be a sulfonic tannic acid with a sulfonic acid group content of 1 mmol/g - 4 mmol/g. For example, the sulfonic acid group content in the sulfonic tannic acid may be 1 mmol/g, 1.25 mmol/g, 1.5 mmol/g, 1.75 mmol/g, 2 mmol/g, 2.25 mmol/g, 2.5 mmol/g, 2.75 mmol/g, 3 mmol/g, 3.25 mmol/g, 3.5 mmol/g, 3.75 mmol/g or 4 mmol/g or in any range composed of the above numerical values.

In a preferred embodiment, the cellulose pretreatment agent may be a sulfonic tannic acid with a sulfonic acid group content of 1.5 mmol/g - 2 mmol/g.

In another particular embodiment, the sulfonic tannic acid may be, but is not limited to a sulfomethyl tannic acid.

It should be understood that the above description of the cellulose pretreatment agent according to the first aspect of the present disclosure is also applicable to the cellulose pretreatment agent used in the step of pretreating cellulose-containing biomass comprised in the method for producing a reducing sugar according to the third aspect of the present disclosure; and the above description of the method for pretreating cellulose according to the second aspect of the present disclosure is also applicable to step (1) of pretreating cellulose-containing biomass comprised in the method for producing a reducing sugar according to the third aspect of the present disclosure. Thus, such content will not be repeated herein for the sake of brevity.

In a particular embodiment, step (1) comprises heating a mixture of the pretreatment agent and the cellulose-containing biomass to a temperature, e.g., from 40°C to 60°C, for a period of time. Preferably, the heating is performed with shaking or stirring, to promote well mixing of the cellulose pretreatment agent with the biomass and temperature uniformity throughout the reaction system.

The cellulose pretreatment in the prior art is typically performed at a relatively high temperature, e.g., from 90°C to 120°C. In contrast, the cellulose pretreatment according to the present disclosure can be performed at a relatively low temperature.

In a preferred embodiment, step (1) comprises heating the mixture of the cellulose pretreatment agent and the cellulose-containing biomass to a temperature from 45°C to 55°C.

In a more preferred embodiment, step (1) comprises heating the mixture of the pretreatment agent and the cellulose-containing biomass to a temperature of 50°C.

In another particular embodiment, the mixture of the pretreatment agent and the cellulose-containing biomass may be maintained at the above-mentioned temperature for 12 h - 48 h, e.g., 24 h.

In another particular embodiment, in step (1), the cellulose pretreatment agent is at a concentration of 0.5 g/L to 20 g/L. For example, the cellulose pretreatment agent may be at a concentration of 0.5 g/L, 1 g/L, 1.5 g/L, 2 g/L, 2.5 g/L, 3 g/L, 3.5 g/L, 4 g/L, 4.5 g/L, 5 g/L, 5.5 g/L, 6 g/L, 6.5 g/L, 7 g/L, 7.5 g/L, 8 g/L, 8.5 g/L, 9 g/L, 9.5 g/L, 10 g/L, 10.5 g/L, 11 g/L, 11.5 g/L, 12 g/L, 12.5 g/L, 13 g/L, 13.5 g/L, 14 g/L, 14.5 g/L, 15 g/L, 15.5 g/L, 16 g/L, 16.5 g/L, 17 g/L, 17.5 g/L, 18 g/L, 18.5 g/L, 19 g/L, 19.5 g/L or 20 g/L, or in any range composed of the above numerical values.

In a particular embodiment, in step (1), the g-C₃N₄/FeOCl Fenton-like composite is at a concentration of 1 g/L to 3 g/L. For example, the g-C₃N₄/FeOCl Fenton-like composite may be at a concentration of 1 g/L, 1.5 g/L, 2 g/L, 2.5 g/L or 3 g/L, or in any range composed of the above numerical values.

Furthermore, an acidic solution, an alkaline solution, or an ionic liquid is usually used as a solvent in the method of pretreating cellulose in the prior art. However, in the cellulose pretreatment according to the present disclosure, water can be used as a solvent with no need to use any acidic/alkaline solution or ionic liquid. As a result, the cost issues associated with acid and base recovery and waste disposal become moot, and there is no need to use any corrosion-resistant container.

Thus, in a particular embodiment, step (1) comprises adding the cellulose pretreatment agent and the cellulose-containing biomass into water to form a mixture of the cellulose pretreatment agent and the cellulose-containing biomass, and then heating the mixture.

After the cellulose-containing biomass is pretreated with the cellulose pretreatment agent, step (2) is performed, namely separating the cellulose-containing biomass from the cellulose pretreatment agent. In the method of producing a reducing sugar according to the present disclosure, since the cellulose pretreatment agent has been separated prior to the enzymatic hydrolysis step, it should be understood that the cellulose pretreatment agent according to the present disclosure is not involved in the subsequent enzymatic hydrolysis step of the cellulose-containing biomass, and thus does not interact with the enzymes used in the enzymatic hydrolysis step.

The separation of the cellulose-containing biomass from the cellulose pretreatment agent can be carried out via filtration or any other means, as long as the cellulose-containing biomass and the cellulose pretreatment agent can be separated from each other. By means of this separation step, the cellulose pretreatment agent can be recovered for reuse.

Furthermore, the cellulose-containing biomass may be milled into fine powder before it is pretreated with the cellulose pretreatment agent, to increase the contact area between the cellulose pretreatment agent and the cellulose-containing biomass, and to promote the pretreatment effect of the cellulose pretreatment agent on the cellulose-containing biomass. However, from the perspective of subsequent separation of the cellulose pretreatment agent from the cellulose-containing biomass, it is preferred not to mill the cellulose-containing biomass into fine powder before the pretreatment.

After the separation of step (2), step (3) is performed, namely subjecting the separated and pretreated cellulose-containing biomass to enzymatic hydrolysis. The cellulase used in step (3) may be, but is not limited to, CTec3, Enzyme Blend, and CTec2.

In addition, the enzymatic hydrolysis in the prior art is typically performed at a relatively high temperature such as above 100°C. In contrast, thanks to the method of pretreating cellulose according to the present disclosure, the subsequent enzymatic hydrolysis step may be performed at room temperature or at a temperature slightly above room temperature.

Thus, in a particular embodiment, the enzymatic hydrolysis step or step (3) is performed at a temperature from 40°C to 60°C. For example, step (3) may be performed at a temperature of 40°C, 45°C, 50°C, 55°C or 60°C or in any temperature range composed of the above numerical values.

In a preferred embodiment, step (3) may be performed at a temperature of 50°C.

Furthermore, the enzymatic hydrolysis in the prior art sometimes requires an ionic liquid as a solvent. In contrast, in the enzymatic hydrolysis according to the present disclosure, a conventional buffer such as a sodium citrate buffer solution can be used as a solvent, and whereby the cost can be significantly reduced and the cellulase activity can be efficiently maintained.

Thus, in a particular embodiment, step (3) may comprise mixing the pretreated cellulose-containing biomass, cellulase and a buffer solution.

In a preferred embodiment, step (3) is performed at a pH of 4-10. For example, step (3) may be performed at pH4, pH4.5, pH5, pH5.5, pH6, pH6.5, pH7, pH7.5, pH8, pH8.5, pH9, pH9.5 or pH10 or in any pH range composed of the above numerical values.

In a more preferred embodiment, step (3) is performed at a pH of 5-7.

In a further more preferred embodiment, step (3) is performed at pH5.5.

Furthermore, it is to be noted that, the pretreated cellulose-containing biomass may be milled into fine powder before it is enzymatically hydrolyzed with cellulase, so as to increase the surface area of the cellulose-containing biomass, and to promote the enzymatic hydrolysis effect of the cellulase on the cellulose-containing biomass.

The pretreatment of the cellulose-containing biomass with the cellulose pretreatment agent according to the present disclosure allows the cellulose structure to become loosened under a mild condition, and in turn allows for an increased efficiency of the subsequent enzymatic hydrolysis of cellulose and a higher yield of the reducing sugar.

Compared with the prior art, the pretreatment according to the present disclosure allows both the cellulose pretreatment and the enzymatic hydrolysis to be performed under a relatively mild condition, and the reaction system of the present disclosure is simpler as no base, acid, inducer or ionic liquid is used in the method of the present disclosure.

In a fourth aspect of the present disclosure, provided is use of the cellulose pretreatment agent according to the first aspect or a g-C₃N₄/FeOCl Fenton-like composite for pretreating cellulose-containing biomass.

As described above, by pretreating the cellulose-containing biomass with an organic acid comprising at least one sulfonic acid group or a carbon-based material, e.g., carbon black or carbon quantum dots, derived from the organic acid comprising at least one sulfonic acid group according to the present disclosure, the sulfonic acid group and the reactive group such as a hydroxyl group (in some cases) on the cellulose pretreatment agent can undergo sufficient chemical reaction with the cellulose under a mild condition, which allows to loosen the cellulose structure and reduce crystallinity degree and the polymerization degree of the cellulose, thereby promoting subsequent hydrolysis by cellulase. The inventors have found that the cellulose-containing biomass pretreated with the cellulose pretreatment agent according to the present disclosure, when being subjected to enzymatic hydrolysis, may produce a reducing sugar at a yield of higher than 80%.

In addition, the inventors have found that pretreatment of the cellulose-containing biomass with the g-C₃N₄/FeOCl Fenton-like composite can not only effectively de-color the cellulose-containing biomass, but also increase the yield of the reducing sugar from the cellulose-containing biomass in the subsequent enzymatic hydrolysis step to a certain extent.

### Examples

The following examples illustrate methods for preparing the cellulose pretreatment agent of the present disclosure and characterization of associated properties thereof. Unless otherwise indicated, the test methods employed herein are conventional, and the test materials used in the following examples were purchased from conventional chemical reagent stores. Unless otherwise defined, all technical and scientific terms used herein have the same meaning as commonly understood by those skilled in the art.

### Example 1: Optimization of pH value for enzymatic hydrolysis

**Enzymatic hydrolysis:** 0.3 g of blue fabric (around 1cm x 1cm) was added into 0.05 M sodium citrate buffer solution (3% solid: liquid ratio) with 120 FPU/g feedstock of cellulase CTec2. The pH of the sodium citrate buffer solution was tuned to a pH of 4-10 using 5% H₂SO₄ solution, 0.8 M NaOH solution or water. The resultant mixture was incubated at 50°C for 96 h with shaking for enzymatic hydrolysis. After the hydrolysis, the mixture was filtered to obtain a reducing sugar solution, which was then measured with DNS test for a reducing sugar yield. The results are given in Table 1. As shown in Table 1, the enzymatic hydrolysis was performed at pH5.5 - pH7 to yield the highest amount of the reducing sugar. pH5.5 was chosen for enzymatic hydrolysis in the subsequent experiments as the reaction system of pH5.5 can be prepared simply by diluting the commercially available sodium citrate buffer.

**Table 1: Reducing sugar yield at different pH for enzymatic hydrolysis**

| pH | Reducing sugar yield (%) |
|---|---|
| 10 | 50 |
| 7 | 51 |
| 5.5 | 62 |
| 5 | 48 |
| 4 | 49 |

### Example 2: Preparation and characterization of sulfomethyl tannic acid

5 g of tannic acid, 45 mL of 0.8 M NaOH solution and 37wt% formaldehyde were sequentially added to a reaction vessel and reacted at a temperature of 70°C for 1.5 h, with the molar ratio of formaldehyde to sodium sulfite to be added in the next step controlled to be 0.8. Sodium sulfite was then added, and the ratio of sodium sulfite (mmol) to tannic acid (g) was controlled to be 1 mmol/g, 1.5 mmol/g, 1.75 mmol/g, 2 mmol/g, and 4 mmol/g. The reaction was then carried out at a temperature of 95°C for 3 h. After the reaction, the resultant products were freeze-dried or oven-dried to obtain the powder, i.e., sulfomethyl tannic acid with a sulfonic acid group content of 1 mmol/g, 1.5 mmol/g, 1.75 mmol/g, 2 mmol/g and 4 mmol/g, respectively.

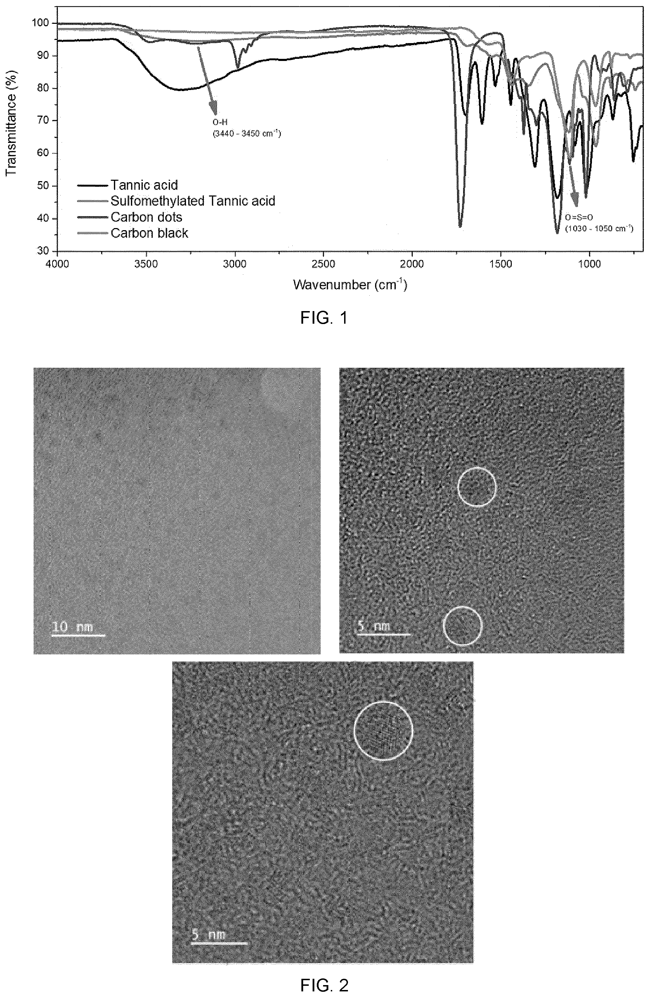

The tannic acid and sulfomethyl tannic acid were tested for infrared spectrum and the results are shown in FIG. 1. As can be seen from FIG. 1, as compared to tannic acid, sulfomethyl tannic acid containing sulfonylmethyl group has an O=S=O vibrational peak at 1030 cm⁻¹ to 1050 cm⁻¹.

### Example 3: Optimization of temperature for pretreatment

**Pretreatment:** 50 mg of freeze-dried sulfomethyl tannic acid with a sulfonic acid group content of 1.75 mmol/g was added into 50 mL of deionized water. 1 g of blue fabric was added into the solution. The resultant mixture was incubated at 40°C, 50°C and 60°C for 24 h, respectively. The pretreated fabric was then filtered, washed with water and dried at 70°C overnight. The dried fabric was milled into fine powder and used as the feedstock for enzymatic hydrolysis.

**Enzymatic hydrolysis:** 0.3 g of milled blue fabric was added into 0.05 M sodium citrate buffer solution (3% solid: liquid ratio) with 120 FPU/g feedstock of cellulase CTec2. The resultant mixture was incubated at 50°C for 96 h with shaking for enzymatic hydrolysis. After the hydrolysis, the mixture was filtered to obtain a reducing sugar solution, which was then measured with DNS test for a reducing sugar yield. The results are given in Table 2. As shown in Table 2, the reducing sugar yield was high, though the pretreatment was performed at a relatively low temperature of 40°C - 50°C.

**Table 2: Reducing sugar yield at different temperatures for pretreatment**

| Temperature (°C) | Reducing sugar yield (%) |
|---|---|
| 40 | 73 |
| 50 | 79 |
| 60 | 67 |

### Example 4: Enzymatic hydrolysis of pretreated blue fabric

**Pretreatment:** 25 mg, 50 mg, 100 mg, 150 mg, 250 mg, 500 mg, 750 mg, 1000 mg of sulfomethyl tannic acid (freeze-dried and oven-dried) with a sulfonic acid group content of 1 mmol/g, 1.75 mmol/g and 4 mmol/g, respectively, were added into 50 mL of deionized water to reach a concentration of 0.5 g/L, 1 g/L, 2 g/L, 3 g/L, 5 g/L, 10 g/L, 15 g/L and 20 g/L sulfomethyl tannic acid. 1 g of blue fabric was added into the resultant solution. The resultant mixture was incubated at 50°C for 24 h. The pretreated fabric was then filtered, washed with water and dried at 70°C overnight. The dried fabric was milled into fine powder and used as the feedstock for enzymatic hydrolysis.

**Enzymatic hydrolysis:** 0.3 g of the milled blue fabric was added into 0.05 M sodium citrate buffer solution (3% solid: liquid ratio, pH5.5) with 120 FPU/g feedstock of cellulase CTec2. The resultant mixture was incubated at 50°C for 96 h with shaking for enzymatic hydrolysis. After the hydrolysis, the mixture was filtered to obtain a reducing sugar solution, which was then measured with DNS test for a reducing sugar yield. The results are given in Table 3.

**Table 3: Reducing sugar yield of the blue fabric pretreated with different concentrations of sulfomethyl tannic acid by enzymatic hydrolysis**

| Sulfonic acid group content (mmol/g) | Drying method | Concentration of the pretreatment agent (g/L) | Reducing sugar yield (%) |
|---|---|---|---|
| 1 | Freeze-dried | 50 | 63 |
| | | 100 | 67 |
| | | 150 | 66 |
| | | 250 | 67 |
| | | 500 | 67 |
| | | 750 | 62 |
| 1.75 | | 25 | 68 |
| | | 50 | 79 |
| | | 100 | 76 |
| | | 150 | 75 |
| | | 250 | 72 |
| | | 500 | 69 |
| | | 750 | 72 |
| | | 1000 | 69 |
| 4 | | 50 | 64 |
| | | 100 | 65 |
| | | 150 | 68 |
| | | 250 | 66 |
| | | 500 | 67 |
| | | 750 | 66 |
| 1.75 | oven-dried | 50 | 83 |
| | | 100 | 81 |
| | | 150 | 89 |
| 0 * | | 0 | 62 |

| | | | |
|---|---|---|---|
| * denotes the control, which was subjected to the same enzymatic hydrolysis step but was not pretreated. | | | |

The results show that the fabric pretreated with the cellulose pretreatment agent of the present disclosure could achieve a higher reducing sugar yield when being subjected to enzymatic hydrolysis, as compared to the control.

### Example 5: Preparation of carbon black with SO₃H group and related pretreatment and enzymatic hydrolysis

**Preparation:** 1 g of sulfomethyl tannic acid with a sulfonic acid group content of 1.75 mmol/g and 4 mmol/g, respectively, were placed in a crucible, heated in a muffle furnace at 300°C for 6 h, and then cooled naturally to room temperature, thereby obtaining carbon black with a SO₃H group content of 1.75 mmol/g and 4 mmol/g.

**Characterization:** The prepared carbon black was tested for infrared spectrum and the result is also shown in FIG. 1. As can be seen from FIG. 1, the prepared carbon black has an O=S=O vibrational peak at 1030 cm⁻¹ to 1050 cm⁻¹, indicating that sulfonic acid groups are present on the carbon black.

**Pretreatment:** 50 mg (at a final concentration of 1 g/L) and 250 mg (at a final concentration of 5 g/L) of the carbon black with a sulfonic acid group content of 1 mmol/g, 1.5 mmol/g, 1.75 mmol/g, 2 mmol/g and 4 mmol/g, respectively, were added into 50 mL of deionized water. 1 g of blue fabric was added into the solution. The resultant mixture was incubated at 50°C for 24 h. The pretreated fabric was then filtered, washed with water and dried at 70°C overnight. The dried fabric was milled into fine powder and used as the feedstock for enzymatic hydrolysis.

**Enzymatic hydrolysis:** 0.3 g of milled fabric was added into 0.05 M sodium citrate buffer solution (3% solid: liquid ratio, pH5.5) with 120FPU/g feedstock of cellulase CTec2. The resultant mixture was incubated at 50°C for 96 hours with shaking for enzymatic hydrolysis. After the hydrolysis, the mixture was filtered to obtain a reducing sugar solution, which was then measured with DNS test for the reducing sugar yield. The results are given in Table 4.

**Table 4: Reducing sugar yield of blue fabric pretreated with different carbon black by enzymatic hydrolysis**

| Sulfonic acid group content (mmol/g) | Concentration of the pretreatment agent (g/L) | Reducing sugar yield (%) |
|---|---|---|
| 1.0 | 50 | 64 |
| | 250 | 62 |
| 1.5 | 50 | 64 |
| | 250 | 64 |
| 1.75 | 50 | 66 |
| | 250 | 67 |
| 4.0 | 50 | 64 |
| | 250 | 70 |
| 2.0 | 50 | 65 |
| 0* | 0 | 62 |

| | | |
|---|---|---|
| * denotes the control, which was subjected to the same enzymatic hydrolysis step but was not pretreated. | | |

As shown in Table 4, as compared to the control, the carbon black of the present disclosure could effectively pretreat the cellulose-containing biomass, so that the pretreated cellulose-containing biomass, even when being subjected to enzymatic hydrolysis under a mild condition, could still achieve a higher reducing sugar yield.

### Example 6: Preparation of carbon quantum dots from citric acid and sulfomethyl tannic acid and related pretreatment and enzymatic hydrolysis

**Preparation:** 10 mmol of citric acid and 0.01 mmol of sulfomethyl tannic acid with a sulfonic acid group content of 4.0 mmol/g were dissolved in 20 ml of ethanol. The solutions were filtered into a 50 ml Teflon-lined vessel. The vessel was placed into a stainless-steel autoclave at 160°C for 5 h in an oven. After the reaction, the autoclave was cooled at room temperature. The resultant solution was dried to obtain the carbon quantum dots.

**Characterization:** The prepared carbon quantum dots were tested for infrared spectrum and the result is also shown in FIG. 1. As can be seen from FIG. 1, similar to carbon black or sulfomethyl tannic acid, the prepared carbon quantum dots showed an O=S=O vibrational peak at 1030 cm⁻¹ to 1050 cm⁻¹, indicating the presence of sulfonic acid group on the carbon quantum dots.

Further, a transmission electron microscope (TEM) image of the prepared carbon quantum dots is provided, as shown in FIG. 2, and the carbon quantum dots obtained by the above method were in the size range of 2 nm - 5 nm.

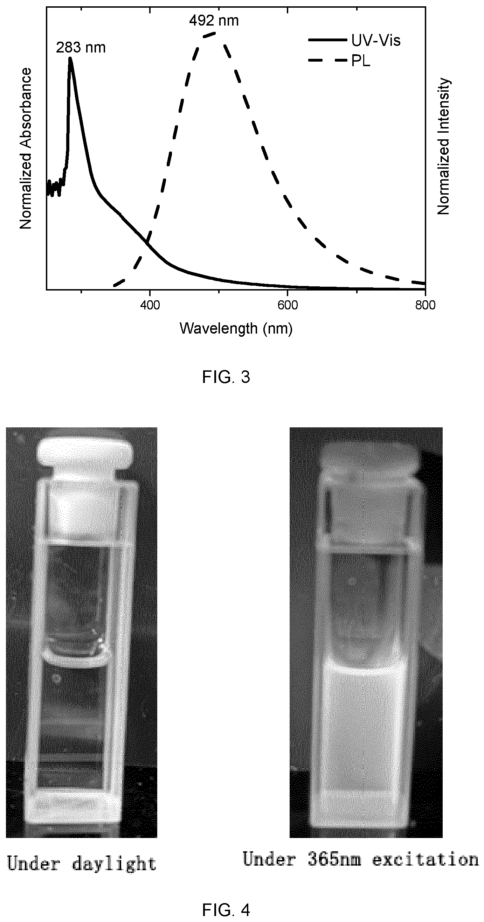

The prepared carbon quantum dots were further tested for photoluminescent properties, and the results are shown in FIGs. 3 and 4.

**Pretreatment:** 50 mg (at a final concentration of 1 g/L) and 500 mg (at a final concentration of 10 g/L) of carbon quantum dots with a sulfonic acid group content of 4.0 mmol/g were added into 50 mL of deionized water. 1g of blue fabric was added into the resultant solution. The resultant mixture was incubated at 50°C for 24 h. The pretreated fabric was then filtered, washed with water and dried at 70°C overnight. The dried fabric was milled into small powder and used as the feedstock for enzymatic hydrolysis.

**Enzymatic hydrolysis:** 0.3 g of milled fabric was added into 0.05 M sodium citrate buffer solution (3% solid: liquid ratio, pH5.5) with 120 FPU/g feedstock of cellulase CTec2. The resultant mixture was incubated at 50°C for 96 h with shaking for enzymatic hydrolysis. After the hydrolysis, the mixture was filtered to obtain the reducing sugar solution, which was then measured with DNS test for the reducing sugar yield. The results are given in Table 5.

**Table 5: Reducing sugar yield of blue fabric pretreated with different concentrations of carbon quantum dots by enzymatic hydrolysis**

| Sulfonic acid group content (mmol/g) | Concentration of the pretreatment agent (g/L) | Reducing sugar yield (%) |
|---|---|---|
| 4.0 | 1 | 65 |
| | 10 | 65 |
| 0* | 0 | 62 |

| | | |
|---|---|---|
| * denotes the control, which was subjected to the same enzymatic hydrolysis step but was not pretreated. | | |

As shown in Table 5, as compared to the control, the carbon quantum dots of the present disclosure could effectively pretreat the cellulose-containing biomass, and the pretreated cellulose-containing biomass, even when being subjected to enzymatic hydrolysis under a mild condition, could still achieve a higher reducing sugar yield.

### Example 7: Preparation of carbon quantum dots from citric acid and metanilic acid and related pretreatment and enzymatic hydrolysis

**Preparation:** 1.728 g of citric acid, 0.7 g of metanilic acid and 600 µL of ethylenediamine were dissolved in 20 mL of dimethylformamide (DMF). The resultant solution was transferred to a 50 mL Teflon-lined vessel. The vessel was then placed into a stainless-steel autoclave for reaction at 160°C for 5 h in an oven. After the reaction, the autoclave was cooled at room temperature. The resultant solution was dried to obtain the carbon quantum dots with a sulfonic acid group content of 5.6 mmol/g.

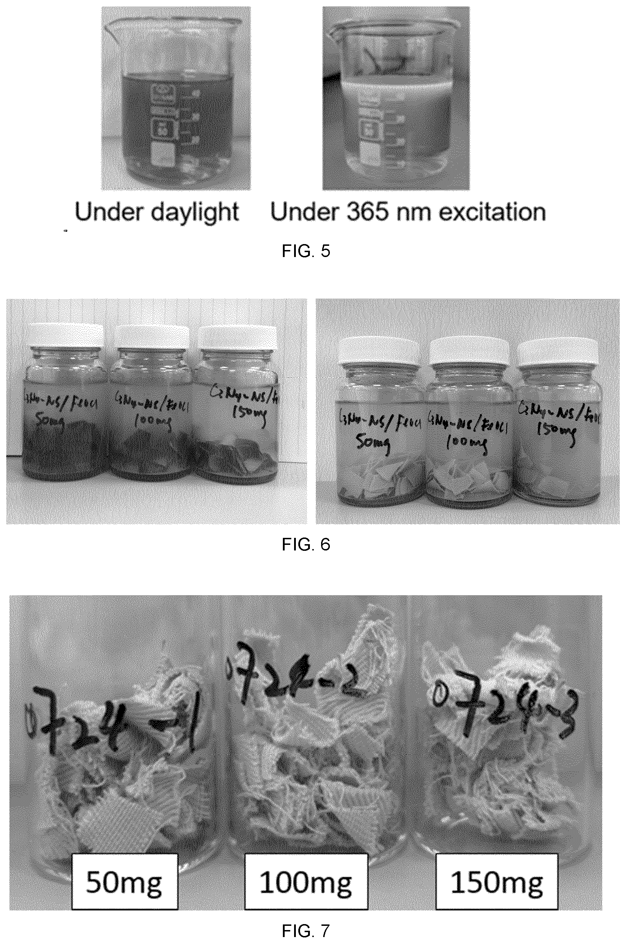

**Characterization:** The prepared carbon quantum dots were tested for photoluminescent properties and the results are shown in FIG. 5. It can be seen that the solution emitted tea color under daylight and blue fluorescence under 365 nm excitation, which indicates that the carbon quantum dots have been successfully prepared.

**Pretreatment:** 50 mg (at a final concentration of 1 g/L), 250 mg (at a final concentration of 5 g/L) and 500 mg (at a final concentration of 10 g/L) of the carbon quantum dots prepared in the above steps were added into 50 mL of deionized water. 1 g of blue fabric was added into the resultant solution. The resultant mixture was incubated at 50°C for 24 h. The pretreated fabric was then filtered, washed with water and dried at 70°C overnight. The dried fabric was milled into fine powder and used as the feedstock for enzymatic hydrolysis.

**Enzymatic hydrolysis:** 0.3 g of milled fabric was added into 0.05 M sodium citrate buffer solution (3% solid: liquid ratio, pH5.5) with 120 FPU/g feedstock of cellulase CTec2. The resultant mixture was incubated at 50°C for 96 h with shaking for enzymatic hydrolysis. After the hydrolysis, the mixture was filtered to obtain a reducing sugar solution, which was then measured with DNS test for the reducing sugar yield. The results are given in Table 6.

**Table 6: Reducing sugar yield of blue fabric pretreated with different concentrations of carbon quantum dots by enzymatic hydrolysis**

| Sulfonic acid group content (mmol/g) | Concentration of the pretreatment agent (g/L) | Reducing sugar yield (%) |
|---|---|---|
| 5.6 | 1 | 68 |
| | 5 | 79 |
| | 10 | 81 |
| 0* | 0 | 62 |

| | | |
|---|---|---|
| * denotes the control, which was subjected to the same enzymatic hydrolysis step but was not pretreated. | | |

As shown in Table 6, as compared to the control, the carbon quantum dots of the present disclosure could effectively pretreat the cellulose-containing biomass, and the pretreated cellulose-containing biomass, even when being subjected to enzymatic hydrolysis under a mild condition, could still achieve a higher reducing sugar yield.

### Example 8: Pretreatment with Fenton-like composite

**Preparation:** Melamine (10.002 g) was calcined at 500°C for 2 h at a heating rate of Δ10°C/min, then 520°C for 2 h at a heating rate of Δ10°C/min. The bulk C₃N₄ was milled into fine powder and calcined at 520°C for 2 h at a heating rate of Δ10°C/min, thereby obtaining the C₃N₄-nanosheet as the product. 0.45 g of the C₃N₄-nanosheet was subjected to ultrasound treatment in 100 mL H₂O to obtain a suspension. 0.13 g FeCl₃·6H₂O in 20 mL H₂O was added dropwise into the suspension. The resultant mixture was stirred for 4 h, and then completely dried in an oven at 70°C. The obtained powder was then calcined at 250°C for 2 h at a heating rate of Δ10°C/min, thereby obtaining the C₃N₄-nanosheet/FeOCl Fenton-like composite.

**Pretreatment:** 50 mg, 100 mg or 150 mg of the C₃N₄-nanosheet/FeOCl Fenton-like composite and 384 mg oxone were added into 50 mL of deionized water. 1 g blue fabric was added to the mixture. The resultant mixture was incubated at 50°C for 24 h. The fabric was then washed with DI water and dried in an oven at 70°C overnight.

FIG. 6 shows the comparison of the blue fabrics before and after incubation with the pretreatment agent, in which the left panel is a photo of the blue fabric before pretreatment, and the right panel is a photo of the blue fabric after pretreatment. FIG. 7 shows the oven-dried fabric. It can be seen from the two figures that the blue pigment was successfully removed from the fabric pretreated with the g-C₃N₄/FeOCl Fenton-like composite.

**Enzymatic hydrolysis:** 0.3 g fabric was added into 0.05 M sodium citrate buffer solution (3% solid: liquid ratio) with 40 FPU/g feedstock of cellulase CTec2. The resultant mixture was incubated at 50°C for 96 h with shaking for enzymatic hydrolysis. After the hydrolysis, the mixture was filtered to obtain a reducing sugar solution, which was then measured with DNS test for the reducing sugar yield.

**Table 7: Reducing sugar yield of blue fabric pretreated with different concentrations of the g-C₃N₄/FeOCl Fenton-like composite**

| g-C₃N₄/FeOCl (g/L) | Reducing sugar yield (%) |
|---|---|
| 1 | 48 |
| 2 | 47 |
| 3 | 51 |
| 0* | 40 |

| | |
|---|---|
| * denotes the control, which was subjected to the same enzymatic hydrolysis step but was not pretreated. | |

As can be seen from Table 7, the use of the C₃N₄-nanosheet/FeOCl Fenton-like composite of the present disclosure significantly increased the yield of the reducing sugar.

Those skilled in the art, in light of the present disclosure, will appreciate that many changes can be made in the specific embodiments which are disclosed herein and alike or similar results can still be obtained without departing from or exceeding the spirit or scope of the disclosure. Those skilled in the art will further understand that any properties reported herein represent properties that are routinely measured and may be obtained by multiple different methods. The methods described herein represent one such method and other methods may be utilized without exceeding the scope of the present disclosure.

The foregoing description of various forms of the disclosure has been presented for purposes of illustration and description. It is not intended to be exhaustive or to limit the disclosure to the precise forms disclosed. Numerous modifications or variations are possible in light of the above teachings. The forms discussed were chosen and described to provide the best illustration of the principles of the disclosure and its practical application to thereby enable one of ordinary skill in the art to utilize the disclosure in various forms and with various modifications as are suited to the particular use contemplated. All such modifications and variations are within the scope of the disclosure as determined by the appended claims when interpreted in accordance with the breadth to which they are fairly, legally, and equitably entitled.

## Claims

1. A cellulose pretreatment agent, wherein the cellulose pretreatment agent is an organic acid comprising at least one sulfonic acid group or a carbon-based material derived from the organic acid comprising at least one sulfonic acid group.

2. The cellulose pretreatment agent of claim 1, wherein the organic acid comprising at least one sulfonic acid group is sulfonic tannic acid and/or metanilic acid.

3. The cellulose pretreatment agent of claim 2, wherein the cellulose pretreatment agent is a sulfonic tannic acid with a sulfonic acid group content of 1 mmol/g - 4 mmol/g.

4. The cellulose pretreatment agent of any one of claims 1-3, wherein the carbon-based material derived from the organic acid comprising at least one sulfonic acid group is carbon black or carbon quantum dots.

5. A method for pretreating cellulose, comprising a step of (1) pretreating cellulose-containing biomass with a cellulose pretreatment agent, wherein the cellulose pretreatment agent is an organic acid comprising at least one sulfonic acid group, a carbon-based material derived from the organic acid comprising at least one sulfonic acid group, or a g-C₃N₄/FeOCl Fenton-like composite.

6. A method for producing a reducing sugar, comprising steps of:
(1) pretreating cellulose-containing biomass with a cellulose pretreatment agent, wherein the cellulose pretreatment agent is an organic acid comprising at least one sulfonic acid group, a carbon-based material derived from the organic acid comprising at least one sulfonic acid group, or a g-C₃N₄/FeOCl Fenton-like composite;
(2) separating the pretreated cellulose-containing biomass from the cellulose pretreatment agent; and
(3) subjecting the separated and pretreated cellulose-containing biomass to enzymatic hydrolysis.

7. The method of claim 5 or 6, wherein the organic acid comprising at least one sulfonic acid group is a sulfonic tannic acid and/or a metanilic acid.

8. The method of claim 7, wherein the cellulose pretreatment agent is a sulfonic tannic acid with a sulfonic acid group content of 1 mmol/g - 4 mmol/g.

9. The method of any one of claims 5-8, wherein the carbon-based material derived from the organic acid comprising at least one sulfonic acid group is carbon black or carbon quantum dots.

10. The method of any one of claims 5-9, wherein step (1) comprises heating a mixture of the pretreatment agent and the cellulose-containing biomass to a temperature from 40°C to 60°C, preferably 45°C to 55°C, and more preferably 50°C for 12 h to 48 h, and preferably 24 h.

11. The method of any one of claims 5-10, wherein in step (1), the cellulose pretreatment agent is at a concentration of 0.5 g/L to 20 g/L, and the g-C₃N₄/FeOCl Fenton-like composite is at a concentration of 1 g/L to 3 g/L.

12. The method of any one of claims 6-11, wherein step (3) is performed at a temperature from 40°C to 60°C or 50°C.

13. The method of any one of claims 6-12, wherein step (3) is performed at a pH of 4 - 10, and preferably 5 - 7.

14. Use of the cellulose pretreatment agent of any one of claims 1-4 or a g-C₃N₄/FeOCl Fenton-like composite for pretreating cellulose-containing biomass.

## Amended claims

### Amended claims in accordance with Rule 137(2) EPC.

1. A method for pretreating cellulose, comprising a step of (1) pretreating cellulose-containing biomass with sulfonic tannic acid, metanilic acid, or carbon black or carbon quantum dots derived from sulfonic acid or metanilic acid.

2. The method of claim 1, wherein the sulfonic tannic acid has a sulfonic acid group content of 1 mmol/g - 4 mmol/g.

3. The method of claim 1 or 2, whrein the pretreating comprises heating a mixture of step (1) to a temperature from 40 °C to 60 °C, preferably 45 °C to 55 °C, and more preferably 50 °C fro 12 h to 48 h, and preferably 24 h.

4. The method of any one of claims 1-3, wherein the sulfonic tannic acid, metanilic acid, or carbon black or carbon quantum dots derived from sulfonic tannic acid or metanilic acid is at a concentration of 0.5 g/L to 20 g/L.

5. A method for producing a reducing sugar, comprising steps of:
(1) pretreating cellulose-containing biomass with sulfonic tannic acid, metanilic acid, or carbon black or carbon quantum dots derived from sulfonic acid or metanilic acid;
(2) separating the pretreated cellulose-containing biomass from a mixture of step (1); and
(3) subjecting the separated and pretreated cellulose-containing biomass to enzymatic hydrolysis.

6. The method of claim 5, wherein the sulfonic tannic acid has a sulfonic acid group content of 1 mmol/g - 4 mmol/g.

7. The method of claim 5 or 6, wherein step (1) comprises heating a mixture of step (1) to a temperature from 40°C to 60°C, preferably 45°C to 55°C, and more preferably 50°C for 12 h to 48 h, and preferably 24 h.

8. The method of any one of claims 5-7, wherein in step (1), the sulfonic tannic acid, metanilic acid, or carbon black or carbon quantum dots derived from sulfonic acid or metanilic acid is at a concentration of 0.5 g/L to 20 g/L.

9. The method of any one of claims 5-8, wherein step (3) is performed at a temperature from 40°C to 60°C or 50°C.

10. The method of any one of claims 5-9, wherein step (3) is performed at a pH of 4 - 10, and preferably 5 - 7.

11. Use of sulfonic tannic acid, metanilic acid, or carbon black or carbon quantum dots derived from sulfonic acid or metanilic acidt for pretreating cellulose-containing biomass.
